# EUROPEAN PATENT APPLICATION

(11) **EP 4 834 345 A1**
(43) Date of publication of application: **04.11.2026**
(21) Application number: 25173993.4
(22) Date of filing: 02.05.2025
(51) Int. Cl.: A61K 31/505, A61K 45/06, A61P 35/00

(54) **ETRAVIRINE FOR USE IN THE TREATMENT OF CANCER**

(71) Applicant: Purposeful, Ike, 11251 Athens (GR)
(72) Inventor: Drakakis, Georgios, 11251 Athens (GR); Chomenidis, Charalampos, 11251 Athens (GR); Tsiliki, Georgia, 11251 Athens (GR); Vlachou, Thaleia, 11251 Athens (GR); Michail, Artemis, 11251 Athens (GR); Ntari, Lydia, 11251 Athens (GR)
(74) Representative: Hamm&Wittkopp Patentanwälte PartmbB

(57) **Abstract**

The present invention relates to the use of Etravirine and a pharmaceutical composition containing Etravirine, preferably a tablet, for the treatment of cancer, in particular solid tumors.

## Description

### Technical Field

The present invention relates to Etravirine and a pharmaceutical composition containing this drug for use in preventing or treating cancer. The present invention provides a pharmaceutical composition capable of inhibiting cancer cell growth and proliferation by modulating multiple molecular pathways involved in oncogenesis and tumor progression. The anticancer activity of the pharmaceutical composition of the present invention is demonstrated across a range of tumors, including, but not limited, to colorectal cancer, pancreatic cancer, prostate cancer, lung cancer, glioblastoma, and neuroblastoma.

### Summary of the Invention

Etravirine is an antiretroviral agent used in the treatment of HIV-infection. Its mechanism of action involves direct inhibition of the HIV-1 reverse transcriptase enzyme. Etravirine has a terminal elimination half-life of 41 hours (Havens, 2020) and the adult dosage is 400 mg (200 mg twice daily or 400 mg once daily). It is approved for antiretroviral treatment-experienced children and adolescents aged 2-18 years old and weighing a minimum of 10 kg. It is commonly used as a second-line treatment due to its higher genetic barrier to resistance development. Etravirine is considered as safe and well tolerated for life long administration (Allavena et al., 2016). The drug is extensively metabolised in the liver *via* cytochrome P450 enzymes, particularly CYP3A4, CYP2C9, and CYP2C19. The metabolites formed retain less than 90% of Etravirine's activity (Scholler-Gyure, Kakuda, Raoof, De Smedt, & Hoetelmans, 2009).

The inventors have conducted a comprehensive *in vitro* kinase profiling assay to elucidate the anticancer mechanisms of Etravirine. This high-throughput kinase panel analysis revealed that Etravirine exhibits significant inhibitory activity against a distinct group of serine/threonine and tyrosine kinases known to be critically involved in cancer cell proliferation, survival, and progression.

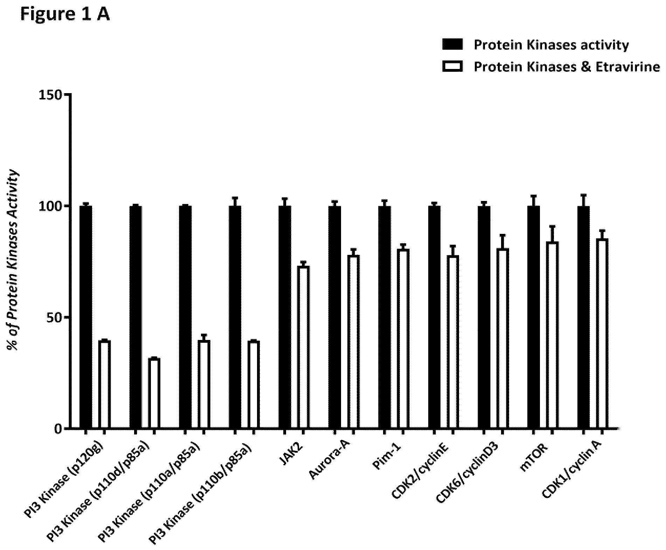

In particular, Etravirine demonstrated downregulation of the following kinases: Phosphoinositide 3-Kinase (PI3K), including the isoforms p120γ (PI3Kγ), p110b/p85α (PI3Kβ), p110δ/p85α (PI3Kδ) and p110α/p85a (PI3Kα), Janus Kinase 2 (JAK2), Aurora Kinase A (Aurora A), Proto-oncogene Serine/Threonine-Protein Kinase Pim-1 (Pim1), Cyclin-Dependent Kinase 2/Cyclin E Complex (CDK2/Cyclin E), Cyclin-Dependent Kinase 6/Cyclin D3 Complex (CDK6/Cyclin D3), the Cyclin-Dependent Kinase 1/Cyclin A Complex (CDK1/Cyclin A) and Mechanistic Target of Rapamycin (mTOR) (Figure 1). These kinases are widely acknowledged to contribute to core oncogenic processes, including cell cycle progression, DNA replication, metabolic regulation, chromosomal stability, and resistance to apoptosis.

Accordingly, Etravirine is suitable for the treatment or prevention of cancer, in particular solid tumors.

### Background to the Invention

The Phosphoinositide 3-Kinases (PI3Ks) constitute a family of lipid kinases that play a pivotal role in intracellular signaling networks regulating cell growth, metabolism, survival, motility, and immune function. Among these, the Class I PI3Ks are particularly relevant in oncogenic signaling and are frequently implicated in the pathogenesis of various human cancers (Baselga, 2011; Fruman et al., 2017; He et al., 2021). PI3Ks are divided in different classes, with the Class I PI3Ks functioning as heterodimers composed of a p110 catalytic subunit and a regulatory one. The PIK3CA, PIK3CB, PIK3CG, and PIK3CD genes encode the catalytic isoforms p110α, p110β, p110γ, and p110δ, respectively (Baselga, 2011).

Class IA PI3Ks (p110α, p110β, p110δ) are typically activated downstream of Receptor Tyrosine Kinases (RTKs), such as EGFR, VEGFR, and FGFR, while Class IB PI3Ks (notably p110γ) are mainly linked to signaling initiated by G protein-coupled receptors (GPCRs) (Miricescu et al., 2020).

Specifically, PI3Kα (p110α) represents one of the most frequently mutated isoforms in human cancer with its activating mutations being commonly noted in early events of malignancies such as breast and colorectal cancer (Berns et al., 2007). PI3Kβ (p110β), encoded by PIK3CB, is extensively expressed, though transforming mutations in PIK3CB are rare. Nonetheless, it may serve compensatory functions within the PI3K network, particularly in cancers exhibiting PTEN mutation, where it becomes a key driver of PI3K signaling. While less frequently mutated than PI3Kα, PI3Kβ remains a viable therapeutic target, especially in tumors with redundant PI3K pathway activation (Baselga, 2011). PI3Kδ (p110δ), encoded by PIK3CD, is physiologically expressed in hematopoietic cells, with an essential role in B cell development, activation, and survival (Fruman et al., 2017). It is activated by Antigen Receptor signaling in B and T lymphocytes and plays a pivotal role in immune regulation. Dysregulation of PI3Kδ has been implicated in hematologic malignancies, particularly chronic lymphocytic leukemia (CLL) and non-Hodgkin's lymphoma (Baselga, 2011). Finally, PI3Kγ (p110γ) is mechanistically differentiated from the other family members, but also plays a key role in oncogenic dysregulations. Under physiological conditions, PI3Kγ is expressed in myeloid-derived cells, where it modulates immune responses. Alterations in PI3Kγ expression have been associated with cancer progression, particularly in colorectal cancer. Interestingly, both over-expression and suppression of PIK3CG have been linked to disease outcomes, reflecting its context-dependent dual role in cancer (Fruman et al., 2017).

Due to the critical role of PI3Ks in human cancer-where they drive tumor initiation, progression, therapeutic resistance, and poor prognosis-numerous cancer therapies have been developed to specifically target PI3K isoforms. As such, inhibition of PI3K isoforms has emerged as a key therapeutic strategy aimed at disrupting oncogenic signaling cascades critical for cell survival, proliferation, metastasis, angiogenesis, and immune evasion (Baselga, 2011).

Janus kinase 2 (JAK2), a member of the JAK family of protein tyrosine kinases (PTKs), is a key intracellular mediator of cytokine and hormone signaling. It plays a central role in the JAK-STAT pathway, which transmits signals from cytokine and growth factor receptors to the cell nucleus (Qian et al., 2011). Once activated, JAK2 phosphorylates specific tyrosine residues on receptors, creating docking sites for STAT proteins, particularly STAT3. These STATs are, then, phosphorylated, dimerize, and move to the nucleus to regulate genes involved in cell growth, differentiation, and immune responses (Aaronson & Horvath, 2002). Aberrant activation of JAK2 has been identified in several cancers, including myeloproliferative neoplasms (Baxter et al., 2005), while increased STAT3 activity is frequently observed in solid tumors (Ayele et al., 2022). This dysregulation can lead to uncontrolled cell signaling, resulting in excessive blood cell production, cell cycle disruption, impaired apoptosis, and genomic instability, all contributing to cancer development. Studies have shown that persistent STAT3 activation promotes tumor initiation and progression by inhibiting apoptosis and enhancing cell proliferation, angiogenesis, invasion, and metastasis (Sikka et al., 2014). Therefore, the identification and development of novel drugs that can target deregulated JAK2/STAT3 activation effectively remains a potential treatment for tumorigenesis.

Aurora kinase A (Aurora-A) is a serine/threonine kinase that plays a pivotal role in regulating key mitotic processes, including centrosome maturation and spindle assembly (Zheng et al., 2023). Mutations on Aurora-A gene, commonly amplifications, result in over-expression of Aurora-A which has been widely observed across multiple cancer types and is strongly associated with tumor progression and therapy resistance. Aurora-A contributes to several oncogenic processes by promoting cell cycle progression, survival, DNA damage repair, and evasion of apoptosis, while also modulating pathways related to autophagy, metastasis, stemness, and immune evasion (Du, Huang, Liu, Li, & Dong, 2021). Inhibiting Aurora-A is significantly associated with disrupting cell cycle progression, inducing mitotic arrest, and impairing cancer cell proliferation and survival. A range of therapeutic strategies has been developed to target Aurora-A beyond conventional kinase inhibition (Du et al., 2021).

PIM-1 is a constitutively active serine/threonine kinase involved in cell proliferation, survival, and apoptosis regulation (Rathi et al., 2021). Frequently over-expressed in both hematologic malignancies and solid tumors, PIM-1 contributes to tumorigenesis and metastatic progression through its interactions with key oncogenic pathways, including Myc, JAK/STAT, and PI3K/Akt (Toth & Warfel, 2021). Furthermore, PIM-1 promotes cell cycle progression, inhibits apoptosis, enhances invasion, and facilitates drug resistance (Rathi et al., 2021; Toth & Warfel, 2021). Due to its central role in cancer biology, PIM-1 is considered a therapeutically actionable target, and multiple inhibitory strategies are being explored to disrupt its oncogenic functions in cancer (Toth & Warfel, 2021).

Cyclin-dependent kinases (CDKs) are a family of serine/threonine protein kinases that regulate cell cycle progression and are also involved in transcription regulation, DNA damage repair, and epigenetic control (Lee et al., 2021). Their activity is primarily controlled through association with cyclins, forming active CDK-cyclin complexes. For instance, CDK2 binds with Cyclin E to form a complex that peaks during the G1/S transition and plays a critical role in cell cycle regulation. Oncogenic activation of this CDK2/Cyclin E complex is frequently observed in human cancers (Fagundes & Teixeira, 2021). Similarly, the CDK6/Cyclin D3 complex is essential for the G0 to G1 phase transition, and its over-expression has been linked to various cancers (Wang et al., 2017). Another important complex, CDK1/Cyclin A, functions at the S/G2 transition of the cell cycle (Zabihi et al., 2023). Cell cycle progression is tightly regulated by checkpoints and the sequential activation of CDKs to ensure proper phase transitions (Zhang et al., 2022). CDKs 1, 2, and 6 play crucial roles in checkpoint passage and show significantly increased activity in cancer cells (Icard et al., 2019). Aberrant cell cycle activity, a hallmark of cancer, is often marked by overexpression of CDKs or cyclins. Dysregulation in CDK expression, caused by genetic or epigenetic alterations, has been reported in over 80% of human cancers (Peyressatre et al., 2015). Consequently, CDKs are considered as promising targets for cancer therapy (Sánchez-Martínez et al., 2019)

Finally, an atypical serine/threonine kinase is the mechanistic target of rapamycin (mTOR) and acts as a central regulator of cellular growth, metabolism and survival. Functionally, mTOR operates through two distinct complexes-mTORC1 and mTORC2-each responsive to unique upstream signals including nutrients, energy status, and growth factors (Mossmann, Park, & Hall, 2018; Murugan, 2019). mTOR is strongly associated with PI3K pathway, as mTORC1, sensitive to rapamycin, integrates amino acid availability and growth factor signaling *via* PI3K/Akt to promote anabolic processes such as protein synthesis, lipid biosynthesis, and nucleotide production, while suppressing autophagy. mTORC2, which is rapamycin-insensitive, regulates cellular metabolism and cytoskeletal organization primarily through activation of AKT (Mossmann et al., 2018). Over-activation of the mTOR pathway-frequently driven by mutations in upstream regulators such as PI3K or loss of PTEN-leads to metabolic reprogramming and uncontrolled tumor growth, establishing mTOR dysregulation as a hallmark of cancer. mTOR signaling supports oncogenesis by promoting biosynthetic pathways, inhibiting catabolic processes, and adapting metabolic flux to meet tumor cell demands. As far as therapeutic strategy is concerned, mTOR inhibition leads to blocking critical pathways that drive tumor cell growth, proliferation, and metabolic adaptation (Miricescu et al., 2020).

Recent experimental evidence generated by the inventors demonstrates that Etravirine exhibits previously unrecognized inhibitory activity against a panel of the oncogenic kinases analysed above. These kinases are well-established drivers of tumor initiation and progression and their inhibition is significantly associated with anti-proliferative, pro-apoptotic, and anti-metastatic effects. The demonstrated ability of Etravirine to simultaneously modulate multiple cancer-related signaling pathways-including PI3K/Akt/mTOR, Aurora-A-dependent mitotic control, PIM-1-mediated survival, and cell cycle regulation-positions it as a novel multi-target (polypharmacological) therapeutic candidate. Given its existing clinical approval and well-characterized pharmacokinetics, Etravirine emerges as a promising repurposed agent for broad-spectrum oncology applications.

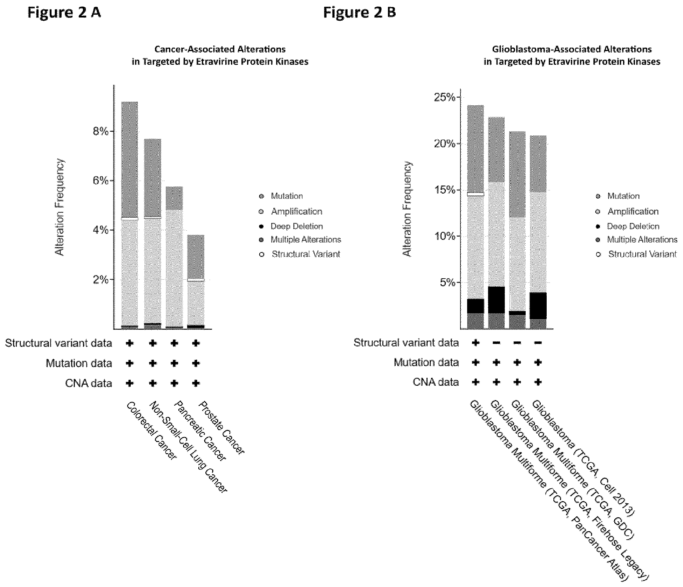

Building upon these findings, the inventors performed an extensive meta-analysis relating the expression pattern of these kinases with various types of human tumors, utilizing publicly available patient cancer genomic datasets, including The Cancer Genome Atlas (TCGA) and cBioPortal. This analysis demonstrated that the aforementioned kinases are recurrently amplified in several high-burden tumor types. The most prevalent amplification events were identified in colorectal cancer, non-small cell lung cancer (NSCLC), pancreatic cancer, prostate cancer and Glioblastoma multiform (GBM) (Figure 2).

Colorectal cancer (CRC) is a leading cause of cancer-related mortality globally, with incidence rising in both aging and younger populations. Approximately 10-20% of cases involve a positive family history, while 5-7% are linked to hereditary syndromes or familial adenomatous polyposis (Dekker et al., 2019; Li et al., 2021). CRC development follows either a conventional adenoma-carcinoma sequence (70-90% of cases), which is characterized by chromosomal instability and mutations in genes such as APC, KRAS, and TP53, leading to microsatellite-stable tumors (Cappell, 2008; Li et al., 2021), or the serrated neoplasia pathway (10-20% of cases), which is associated with BRAF mutations and epigenetic silencing of mismatch repair genes via CpG island methylation, resulting in microsatellite instability (MSI) (Dekker et al., 2019). Treatment selection of CRC depends on disease stage and molecular subtype. Localized CRC is typically managed with surgery and adjuvant chemotherapy, while metastatic disease requires systemic therapy that may include targeted agents (e.g., anti-EGFR or anti-VEGF monoclonal antibodies) or immune checkpoint inhibitors. Despite these advancements, therapeutic resistance, especially from cancer stem cells, remains a significant barrier to durable response (Dekker et al., 2019; Li et al., 2021).

Non-small cell lung cancer (NSCLC) represents about 85% of all lung cancer cases and accounts for an estimated 1.6 million deaths worldwide each year. Tobacco smoking is the primary risk factor, though adenocarcinoma-a major NSCLC subtype-is frequently diagnosed in non-smokers. Environmental exposures and genetic predispositions also contribute to disease risk (Herbst et al., 2018; Mithoowani & Febbraro, 2022). On a molecular level, the pathogenesis of NSCLC is characterized by recurrent genetic alterations, notably in EGFR, KRAS, and ALK. These mutations not only influence tumor behavior, but also serve as predictive biomarkers for response to targeted therapies. The tumor microenvironment also plays a significant role, with PD-L1 expression emerging as a key factor in suppressing anti-tumor immune responses (Herbst et al., 2018). Treatment varies depending on disease stage and molecular profile. In early-stage NSCLC, surgery remains the standard approach, often followed by chemotherapy, while newer evidence supports the use of adjuvant targeted therapy and immunotherapy to reduce recurrence risk (Mithoowani & Febbraro, 2022). For unresectable stage III disease, chemoradiation followed by immunotherapy significantly improves survival. Despite the major advancements in treating NSCLC, the need for new therapies is always prominent, as its high mortality rate underscores the urgent need for more effective and durable treatment options.

Pancreatic cancer, particularly pancreatic ductal adenocarcinoma (PDAC), is a highly aggressive malignancy (3% of all cancers). Its incidence is rising, with over 60,000 new cases annually, and it is projected to become the second-leading cause of cancer mortality by 2030. Most patients are diagnosed at an advanced stage, with only about 10-15% eligible for surgery, but the five-year survival rate remains at around 10% (Park et al., 2021; Kolbeinsson et al., 2023). The pathogenesis of PDAC is driven by a combination of genetic alterations and environmental exposures. The most common mutation is found in the KRAS gene, responsible for over 90% of tumors, typically followed by alterations in tumor suppressor genes such as TP53, SMAD4, and CDKN2A. Germline mutations in BRCA2, BRCA1, PALB2, and ATM are also associated with familial pancreatic cancer syndromes. The tumor microenvironment is highly immunosuppressive, characterized by dense stroma, regulatory immune cells, and low cytotoxic T-cell activity, making PDAC resistant to many immune-based therapies (Park et al., 2021). Treatment strategies are determined primarily by tumor stage at diagnosis. For 10-15% of patients, surgical resection, often a pancreaticoduodenectomy, remains the primary option for treatment, but recurrence remains high, often due to micro-metastases. Patients with borderline resectable or locally advanced tumors typically receive neoadjuvant chemotherapy (Kolbeinsson et al., 2023). In cases of metastatic or unresectable pancreatic cancer, systemic chemotherapy serves as the primary treatment approach (Park et al., 2021; Kolbeinsson et al., 2023). Given its aggressive nature, late diagnosis, and resistance to conventional treatments, pancreatic cancer continues to exhibit a dismal prognosis, necessitating urgent therapeutic innovation.

Prostate cancer is one of the most frequently diagnosed cancers in men and a major contributor to cancer-related deaths globally (Sekhoacha et al., 2022). Although most cases are diagnosed in men over the age of 65, a growing number of younger diagnoses have been observed (Schatten, 2018; Wang et al., 2018). Prostate cancer is driven by a complex combination of inherited genetic mutations (mostly in BRCA1, BRCA2, ATM, and HOXB 1), somatic genomic alterations, and disruptions in key signaling pathways, as well as epigenetic dysregulation and metabolic reprogramming (Sekhoacha et al., 2022; Wang et al., 2018). Tumor progression is tightly linked to androgen receptor (AR) signaling, which regulates prostate cell growth. Treatment selection depends on the stage, risk classification, PSA levels, and patient health. For low-risk, localized disease, active surveillance remains a safe and effective strategy. When curative treatment is required, radical prostatectomy or radiation therapy are standard options, although both may lead to side effects like incontinence and erectile dysfunction (Sekhoacha et al., 2022). In patients with more aggressive disease, Androgen deprivation therapy (ADT) is the primary approach. (Schatten, 2018). Nevertheless, the unmet therapeutic needs remain highly pronounced.

Glioblastoma (GBM) is the most aggressive and frequently diagnosed primary malignant brain tumor in adults. It disproportionately affects older adults, with the average age at diagnosis around 65. Despite advances in multimodal treatment strategies, the prognosis remains extremely poor, with a five-year survival rate of less than 7% (Davis, 2016; Schaff & Mellinghoff, 2023). The molecular mechanisms underlying GBM involve a combination of genetic, epigenetic, and molecular alterations that result in a highly invasive, therapy-resistant tumor. One of the most common molecular features is the amplification or mutation of the epidermal growth factor receptor (EGFR), particularly in the classical subtype of GBM, which leads to uncontrolled cell proliferation. Standard-of-care treatment for glioblastoma involves a combination of maximal safe surgical resection, radiation therapy, and chemotherapy. Targeted therapies and immunotherapeutic approaches have been explored as alternative or complementary options, with some examples being the inhibition of key pathways such as EGFR and PDGFRAs, as well as immunotherapies including immune checkpoint inhibitors and dendritic cell vaccines (Schaff & Mellinghoff, 2023; Lan et al., 2024). Glioblastoma presents a formidable therapeutic challenge due to its aggressive nature, high recurrence rate, and resistance to standard treatments, highlighting a critical need for novel therapeutic strategies.

While cBioPortal analysis identified kinases targeted by Etravirine in specific cancers, as described above, the inventors also included Neuroblastoma in their study due to its mechanistic correlation with Glioblastoma and other neuro-oncological pathways. In particular, the dysregulation of kinase pathways such as Aurora-A, PIM1 and PI3K/Akt is deeply implicated in neural development and survival. Neuroblastoma is the most common extracranial solid tumor in childhood and accounts for about 10% of all pediatric cancers and 15% of pediatric cancer deaths. It has a distinct age distribution, with 90% of cases diagnosed before the age of 10 (Matthay et al., 2016; Whittle et al., 2017). The incidence is approximately 10.5 cases per million children, with minimal geographic variation (Whittle et al., 2017). Neuroblastoma arises from neural crest-derived cells of the developing sympathetic nervous system. The disease is notable for its heterogeneous clinical course: some tumors regress spontaneously, especially in infants, while others exhibit aggressive metastatic behavior, commonly spreading to bone, bone marrow, and liver (Maris et al., 2007). Treatment is risk-based, where low-and intermediate-risk patients, who account for roughly half of cases, are often treated successfully with surgery alone or in combination with low-intensity chemotherapy, achieving long-term survival rates above 90% (Whittle et al., 2017). High-risk neuroblastoma, by contrast, requires intensive multimodal therapy, which typically includes inductive chemotherapy, surgical resection, high-dose chemotherapy with autologous stem cell rescue, radiation, and post-consolidation therapies. Despite these aggressive regimens, the 5-year overall survival for high-risk patients remains below 50% (Maris et al., 2007; Whittle et al., 2017), underscoring the urgent need for more effective and targeted treatments.

All cancer types analysed above are considered as therapeutically challenging malignancies with limited efficacy of current therapy options and an urgent need for novel medical strategies. Through meta-analytical investigations conducted by the inventors, a common mechanistic thread has emerged: these malignancies frequently harbor genetic alterations, particularly amplifications, leading to over-expression of key oncogenic kinases. Notably, these dysregulated kinases fall within the inhibitory spectrum of Etravirine, supporting its potential as a repurposed, multi-target therapeutic anticancer agent.

Amplification of these kinases in cancer cells typically leads to their over-expression and hyperactivation, resulting in the promotion of malignant phenotypes such as uncontrolled proliferation, angiogenesis, metastatic potential, and treatment resistance. The therapeutic inhibition of these kinases by the composition proposed by the inventors represents a well-established and scientifically validated strategy in oncology.

The inventors found that Etravirine's ability to inhibit this unique cluster of oncogenic kinases, several of which are co-amplified in specific cancer types, renders it a promising candidate for therapeutic repurposing in oncology. The dual-targeting capacity of Etravirine-both as a selective kinase modulator and as a previously approved antiviral compound-further supports its translational potential and expedites its clinical development in cancer treatment.

Thus, the present invention provides a pharmaceutical composition comprising Etravirine, optionally in combination with one or more additional agents, for use in the treatment or prevention of solid tumors characterized by amplification or over activation of the previously mentioned kinases. The invention is particularly applicable to cancers including colorectal cancer, non-small cell lung cancer, pancreatic cancer, prostate cancer, glioblastoma and neuroblastoma wherein genomic amplification of PI3K isoforms, JAK2, Aurora A, Pim1, CDK1, CDK2, CDK6 and mTOR has been found. Accordingly, the inventors found that cancer patients exhibiting molecular dysregulation within signaling pathways modulated by these kinases may benefit from treatment with the pharmaceutical composition of the present invention comprising Etravirine.

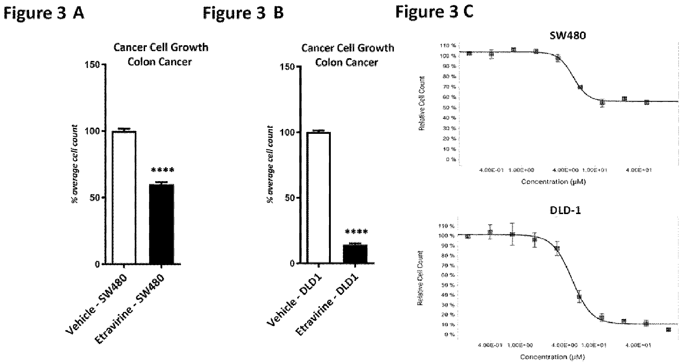

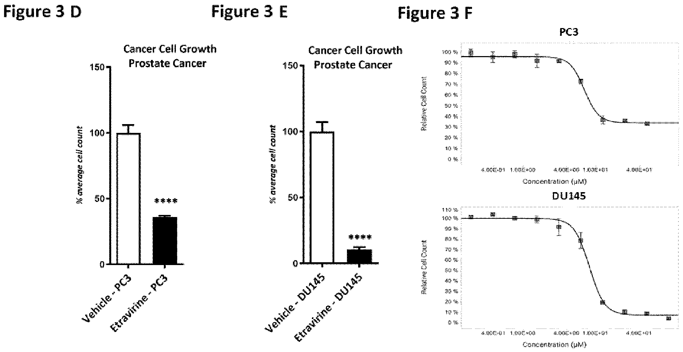

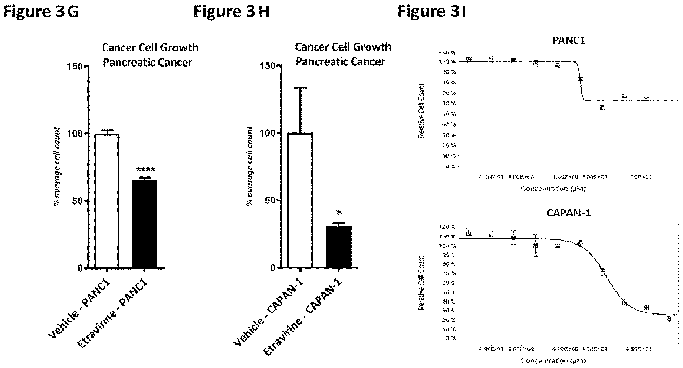

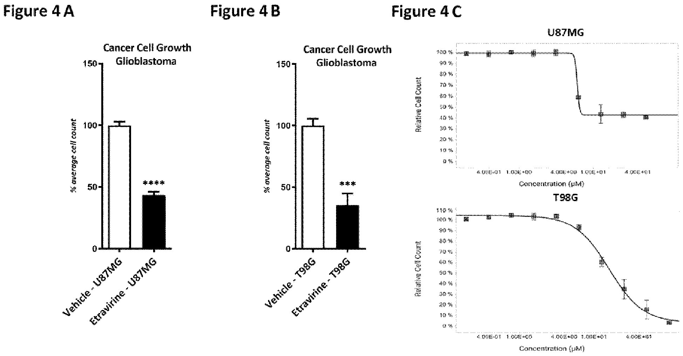

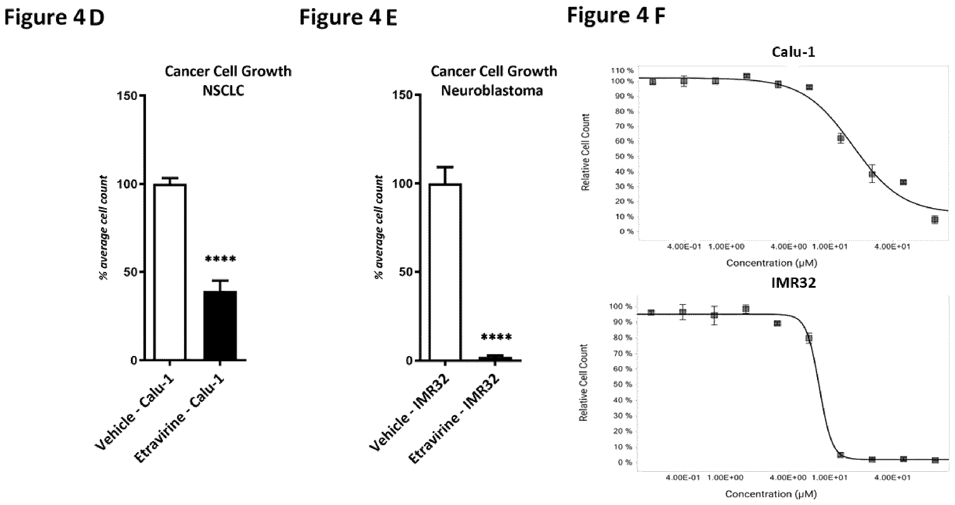

Building upon the bioinformatic evidence of kinase amplification across multiple cancer types (Figure 2), the inventors conducted a comprehensive experimental validation to assess the therapeutic potential of Etravirine on cancer cell growth *in vitro.* Firstly, an in-depth selection process was undertaken to identify representative cancer cell lines across tumor types revealed by inventors' meta-analysis to exhibit recurrent amplification or over-expression of Etravirine-targeted kinases. Based on this integrative bioinformatic and experimental approach, the following cell lines were selected for *in vitro* evaluation: SW-480 and DLD-1 (colon carcinoma), PC3 and DU145 (prostate carcinoma), PANC1 and CAPAN1 (pancreatic carcinoma), U87MG and T98G (Gioblastoma), Calu-1 (non-small-cell lung carcinoma), and IMR32 (Neuroblastoma). These models were specifically chosen due to their high expression levels of the kinases identified as Etravirine targets in kinase inhibition assays, thereby ensuring biological relevance and translational value in evaluating the drug's anti-cancer potential (Figures 3 and 4).

Treatment of these cell lines with Etravirine (administered across a concentration range of 0 to 100 µM) for an incubation period of 3 days, resulted in a marked and consistent reduction in cancer cell viability across all models tested. Notably, a single-dose treatment of Etravirine at 25 µM was sufficient to reduce cell viability by over 55% across all tested cancer cell lines, underscoring its potent and broad-spectrum anti-proliferative activity (Table 1). Quantitative viability assays revealed that Etravirine significantly inhibited proliferation, with further dose-response analysis confirming a dose-dependent inhibitory effect and favorable IC50 values varying between different cancer cell lines (Figures 3 and 4 and Table 1).

**Table 1. Etravirine IC 50 values of all cancer cells lines used in the cancer cell viability in vitro experiment.**

| **Cell line** | **IC 50 (µM)** |
|---|---|
| Calu1 | 19.00 |
| Capan-1 | 18.00 |
| DLD-1 | 5.38 |
| DU145 | 8.54 |
| IMR-32 | 7.93 |
| PANC-1 | >100 |
| PC-3 | 9.23 |
| SW480 | >100 |
| T98G | 16.40 |
| U-87 MG | 6.48 |

These findings experimentally validate the anti-cancer efficacy of Etravirine in tumor types enriched for the dysregulated kinases identified in the initial meta-analysis. Importantly, this data establishes a direct correlation between the molecular profile of kinase dysregulation and cellular sensitivity to Etravirine, thereby supporting the rational repositioning of this compound as a targeted therapeutic strategy in oncology.

### Detailed Description of the Invention

The objective underlying the present invention was the provision of a remedy for the treatment or prevention of cancer. This objective has been solved by the subject matter as defined in the claims.

Embodiments of the invention are described below, by way of example only with reference to and as illustrated in the following figures:
**Figure 1** illustrates the results of an *in vitro* kinase profiling assay performed by the inventors to evaluate the inhibitory effect of Etravirine on a panel of kinases functionally implicated in oncogenesis and tumor progression. Kinase inhibition was measured *in vitro* using a radiometric assay that quantifies the incorporation of radiolabeled phosphate into substrate peptides. Recombinant kinases were incubated with Etravirine, and the resulting phosphorylation activity was assessed to determine the compound's inhibitory effects on individual kinases. Fig. 1A is a bar plot which demonstrates expression levels of kinases implicated in cancer initiation and development before and after Etravirine treatment in an *in vitro* protein kinase activity assay, normalised to the baseline expression of each kinase prior to treatment. The assay was designed to quantify the incorporation of radiolabeled phosphate into specific substrate peptides in the presence and absence of Etravirine. Recombinant forms of each kinase were used to assess the compound's direct inhibitory potential. The data are normalized to baseline phosphorylation activity (set at 100%) for each kinase prior to treatment. Etravirine treatment led to marked reductions in phosphorylation activity across all these targets, indicating its potential to interfere with core cancer signaling networks. These results support the hypothesis that Etravirine may exert multi-target antitumor activity through simultaneous inhibition of several driver kinases involved in cancer initiation and progression.
**Figure 2** demonstrates a Meta-Analysis of Cancer-Related Kinases inhibited by Etravirine in Public Cancer Patients Databases. This analysis provides insights into a comprehensive bioinformatic analysis undertaken by the inventors to investigate the clinical relevance of the kinases inhibited by Etravirine. Publicly available cancer genomic datasets were used to determine the amplification frequency and expression levels of these kinases across multiple tumor types. Specifically, Fig. 2A depicts a gene-level alterations graph in the Etravirine-targeted kinase set across a broad array of human tumors using data sourced from cBioPortal. The analysis in the Pan-Cancer Database reveals that the kinases inhibited by Etravirine are recurrently amplified and/or over-expressed in multiple malignancies, including colorectal cancer, non-small cell lung cancer, pancreatic cancer, prostate cancer, and glioblastoma. These amplification events are strongly associated with poor prognosis, highlighting the clinical relevance and therapeutic significance of targeting these kinases. Fig. 2B specifically focuses on glioblastoma multiform (GBM), a high-grade primary brain tumor with limited treatment options. Using four different integrated datasets from cBioPortal, the inventors demonstrate a high prevalence of gene amplification and over-expression of Etravirine-inhibited kinases within GBM patient samples. This observation supports the potential application of Etravirine in the treatment of glioblastoma, particularly in tumors characterized by dysregulation of these specific kinase pathways .
**Figure 3** demonstrates the inhibitory effects of a 3-day treatment of Etravirine on cancer cell growth across multiple solid tumor types. This figure provides insight into the anti-proliferative properties of Etravirine *in vitro,* as assessed through quantitative cell viability assays in a panel of human cancer cell lines. Fig. 3A and 3B display the quantification of cancer cell growth in the colon carcinoma SW-480 and DLD-1 cell lines, respectively, upon treatment with either vehicle control or Etravirine at a concentration of 25 µM. Fig. 3C presents the dose-response curves and calculated IC50 values for both SW-480 and DLD-1 cell lines, confirming a dose-dependent response to Etravirine treatment. Subsequently, Fig. 3D and 3E evaluate the anti-cancer effects of Etravirine on prostate carcinoma cell lines PC3 and DU145, revealing significant suppression of cell proliferation at a concentration of 25 µM. The corresponding Etravirine dose-response analysis and IC50 determination are depicted in Fig. 3F. Fig. 3G and 3H further expand the analysis to pancreatic carcinoma cell lines PANC1 and CAPAN1, again demonstrating Etravirine-induced growth inhibition at 25 µM concentration. Fig. 3I summarizes the dose-response of Etravirine and IC50 values in these pancreatic cell lines.
**Figure 4** further explores the anti-cancer potential of Etravirine in glioblastoma, lung carcinoma, and neuroblastoma cell models, treated for 3 days. Fig. 4A and 4B show the quantification of cell growth inhibition in glioblastoma U87MG and T98G cell lines following exposure to Etravirine (25 µM), compared to vehicle controls. Etravirine dose-response analysis and IC50 values for these glioblastoma models are provided in Fig. 4C, highlighting the compound's efficacy in this highly aggressive and treatment-resistant cancer type. Fig. 4D illustrates similar growth suppression effects of the same dose of Etravirine (25 µM) in the non-small-cell lung carcinoma Calu-1 cell line, while Fig. 4E focuses on the neuroblastoma IMR32 model. The final Fig. 4F presents the dose-response curves and corresponding IC50 measurements of Etravirine treatment for both Calu-1 and IMR32 cell lines.

These results demonstrate that Etravirine exhibits potent growth-inhibitory activity across diverse tumor types , including cancers with limited therapeutic options. Collectively, these findings underscore the broad-spectrum anti-tumor activity of Etravirine *in vitro,* supporting its potential repurposing as an anti-cancer agent.

### References

Aaronson, D. S., & Horvath, C. M. (2002). A road map for those who don't know JAK-STAT. Science (New York, N.Y.), 296(5573), 1653-1655. https://doi.org/10.1126/SCIENCE.1071545
Ayele, T. M., Muche, Z. T., Teklemariam, A. B., Kassie, A. B., & Abebe, E. C. (2022). Role of JAK2/STAT3 Signaling Pathway in the Tumorigenesis, Chemotherapy Resistance, and Treatment of Solid Tumors: A Systemic Review. Journal of Inflammation Research, 15, 1349-1364. https://doi.org/10.2147/JIR.S353489
Baselga, J. (2011). Targeting the phosphoinositide-3 (PI3) kinase pathway in breast cancer. Oncologist, 16 Suppl 1, 12-19. https://doi.org/10.1634/theoncologist.2011-S1-12
Baxter, E. J., Scott, L. M., Campbell, P. J., et al. (2005). Acquired mutation of the tyrosine kinase JAK2 in human myeloproliferative disorders. Lancet, 365(9464), 1054-1061. https://doi.org/10.1016/S0140-6736(05)71142-9
Berns, K., Horlings, H. M., Hennessy, B. T., et al. (2007). A functional genetic approach identifies the PI3K pathway as a major determinant of trastuzumab resistance in breast cancer. Cancer Cell, 12(4), 395-402. https://doi.org/10.1016/j.ccr.2007.08.030
Cappell, M. S. (2008). Pathophysiology, Clinical Presentation, and Management of Colon Cancer. In Gastroenterology Clinics of North America (Vol. 37, Issue 1, pp. 1-24). https://doi.org/10.1016/j.gtc.2007.12.002
Davis, M. E. (2016). Glioblastoma: Overview of disease and treatment. Clinical Journal of Oncology Nursing, 20(5), 1-8. https://doi.org/10.1188/16.CJON.S1.2-8
Dekker, E., Tanis, P. J., Vleugels, J. L. A., Kasi, P. M., & Wallace, M. B. (2019). Colorectal cancer. In www.thelancet.com. www.thelancet.com
Du, R., Huang, C., Liu, K., Li, X., & Dong, Z. (2021). Targeting AURKA in Cancer: molecular mechanisms and opportunities for Cancer therapy. Molecular Cancer, 20(1), 15. https://doi.org/10.1186/s12943-020-01305-3
Fagundes, R., & Teixeira, L. K. (2021). Cyclin E/CDK2: DNA Replication, Replication Stress and Genomic Instability. Frontiers in Cell and Developmental Biology, 9, 774845. https://doi.org/10.3389/FCELL.2021.774845
Fruman, D. A., Chiu, H., Hopkins, B. D., et al. (2017). The PI3K Pathway in Human Disease. Cell, 170(4), 605-635. https://doi.org/10.1016/j.cell.2017.07.029
He, Y., Sun, M. M., Zhang, G. G., et al. (2021). Targeting PI3K/Akt signal transduction for cancer therapy. Signal Transduction and Targeted Therapy, 6(1), 425. https://doi.org/10.1038/s41392-021-00828-5
Herbst, R. S., Morgensztern, D., & Boshoff, C. (2018). The biology and management of non-small cell lung cancer. In Nature (Vol. 553, Issue 7689, pp. 446-454). Nature Publishing Group. https://doi.org/10.1038/nature25183
Icard, P., Fournel, L., Wu, Z., Alifano, M., & Lincet, H. (2019). Interconnection between Metabolism and Cell Cycle in Cancer. Trends in Biochemical Sciences, 44(6), 490-501. https://doi.org/10.1016/J.TIBS.2018.12.007
Kolbeinsson, H. M., Chandana, S., Wright, G. P., & Chung, M. (2023). Pancreatic Cancer: A Review of Current Treatment and Novel Therapies. In Journal of Investigative Surgery (Vol. 36, Issue 1). Taylor and Francis Ltd. https://doi.org/10.1080/08941939.2022.2129884
Lan, Z., Li, X., & Zhang, X. (2024). Glioblastoma: An Update in Pathology, Molecular Mechanisms and Biomarkers. In International Journal of Molecular Sciences (Vol. 25, Issue 5). Multidisciplinary Digital Publishing Institute (MDPI). https://doi.org/10.3390/ijms25053040
Lee, J. C., Hong, K. H., Becker, A., et al. (2021). Tetrahydroindazole inhibitors of CDK2/cyclin complexes. European Journal of Medicinal Chemistry, 214, 113232. https://doi.org/10.1016/J.EJMECH.2021.113232
Li, J., Ma, X., Chakravarti, D., Shalapour, S., & Depinho, R. A. (2021). Genetic and biological hallmarks of colorectal cancer. https://doi.org/10.1101/gad.348226
Maris, J. M., Hogarty, M. D., Bagatell, R., & Cohn, S. L. (2007). Neuroblastoma. Lancet (London, England), 369(9579), 2106-2120. https://doi.org/10.1016/S0140-6736(07)60983-0
Matthay, K. K., Maris, J. M., Schleiermacher, G., Nakagawara, A., Mackall, C. L., Diller, L., & Weiss, W. A. (2016). Neuroblastoma. Nature Reviews Disease Primers, 2. https://doi.org/10.1038/nrdp.2016.78
Miricescu, D., Totan, A., Stanescu, S., et al. (2020). PI3K/AKT/mTOR Signaling Pathway in Breast Cancer: From Molecular Landscape to Clinical Aspects. International Journal of Molecular Sciences, 22(1). https://doi.org/10.3390/ijms22010173
Mithoowani, H., & Febbraro, M. (2022). Non-Small-Cell Lung Cancer in 2022: A Review for General Practitioners in Oncology. In Current Oncology (Vol. 29, Issue 3, pp. 1828-1839). MDPI. https://doi.org/10.3390/curroncol29030150
Mossmann, D., Park, S., & Hall, M. N. (2018). mTOR signalling and cellular metabolism are mutual determinants in cancer. Nature Reviews Cancer, 18(12), 744-757. https://doi.org/10.1038/s41568-018-0074-8
Murugan, A. K. (2019). mTOR: Role in cancer, metastasis and drug resistance. Seminars in Cancer Biology, 59, 92-111. https://doi.org/10.1016/j.semcancer.2019.07.003
Park, W., Chawla, A., & O'Reilly, E. M. (2021). Pancreatic Cancer: A Review. In JAMA - Journal of the American Medical Association (Vol. 326, Issue 9, pp. 851-862). American Medical Association. https://doi.org/10.1001/jama.2021.13027
Peyressatre, M., Prével, C., Pellerano, M., & Morris, M. C. (2015). Targeting cyclin-dependent kinases in human cancers: from small molecules to Peptide inhibitors. Cancers, 7(1), 179-237. https://doi.org/10.3390/CANCERS7010179
Qian, C. J., Yao, J., & Si, J. M. (2011). Nuclear JAK2: form and function in cancer. Anatomical Record, 294(9), 1446-1459. https://doi.org/10.1002/AR.21443
Rathi, A., Kumar, D., Hasan, G. M., et al. (2021). Therapeutic targeting of PIM KINASE signaling in cancer therapy: Structural and clinical prospects. Biochimica et Biophysica Acta - General Subjects, 1865(11), 129995. https://doi.org/10.1016/j.bbagen.2021.129995
Sánchez-Martínez, C., Lallena, M. J., Sanfeliciano, S. G., & de Dios, A. (2019). Cyclin dependent kinase (CDK) inhibitors as anticancer drugs: Recent advances (2015-2019). Bioorganic & Medicinal Chemistry Letters, 29(20), 126637. https://doi.org/10.1016/J.BMCL.2019.126637
Schaff, L. R., & Mellinghoff, I. K. (2023). Glioblastoma and Other Primary Brain Malignancies in Adults: A Review. In JAMA (Vol. 329, Issue 7, pp. 574-587). American Medical Association. https://doi.org/10.1001/jama.2023.0023
Schatten, H. (2018). Brief overview of prostate cancer statistics, grading, diagnosis and treatment strategies. In Advances in Experimental Medicine and Biology (Vol. 1095, pp. 1-14). Springer New York LLC. https://doi.org/10.1007/978-3-319-95693-0_1
Sekhoacha, M., Riet, K., Motloung, P., Gumenku, L., Adegoke, A., & Mashele, S. (2022). Prostate Cancer Review: Genetics, Diagnosis, Treatment Options, and Alternative Approaches. In Molecules (Vol. 27, Issue 17). MDPI. https://doi.org/10.3390/molecules27175730
Sikka, S., Surana, R., Dai, X., et al. (2014). Targeting the STAT3 signaling pathway in cancer: Role of synthetic and natural inhibitors. Biochimica et Biophysica Acta (BBA) - Reviews on Cancer, 1845(2), 136-154. https://doi.org/10.1016/J.BBCAN.2013.12.005
Toth, R. K., & Warfel, N. A. (2021). Targeting PIM Kinases to Overcome Therapeutic Resistance in Cancer. Molecular Cancer Therapeutics, 20(1), 3-10. https://doi.org/10.1158/1535-7163.MCT-20-0535
Wang, G., Zhao, D., Spring, D. J., & Depinho, R. A. (2018). Genetics and biology of prostate cancer. https://doi.org/10.1101/gad.315739
Wang, H., Nicolay, B. N., Chick, J. M., et al. (2017). The metabolic function of cyclin D3-CDK6 kinase in cancer cell survival. Nature, 546(7658), 426. https://doi.org/10.1038/NATURE22797
Whittle, S. B., Smith, V., Doherty, E., Zhao, S., McCarty, S., & Zage, P. E. (2017). Overview and recent advances in the treatment of neuroblastoma. In Expert Review of Anticancer Therapy (Vol. 17, Issue 4, pp. 369-386). Taylor and Francis Ltd. https://doi.org/10.1080/14737140.2017.1285230
Zabihi, M., Lotfi, R., Yousefi, A. M., & Bashash, D. (2023). Cyclins and cyclin-dependent kinases: from biology to tumorigenesis and therapeutic opportunities. Journal of Cancer Research and Clinical Oncology, 149(4), 1585-1606. https://doi.org/10.1007/S00432-022-04135-6
Zhang, C., Wang, X., & Zhang, C. (2022). Icaritin inhibits CDK2 expression and activity to interfere with tumor progression. iScience, 25(9). https://doi.org/10.1016/J.ISCI.2022.104991
Zheng, D., Li, J., Yan, H., et al. (2023). Emerging roles of Aurora-A kinase in cancer therapy resistance. Acta Pharmaceutica Sinica B, 13(7), 2826-2843. https://doi.org/10.1016/j.apsb.2023.03.01

## Claims

1. Etravirine for use in the treatment or prevention of cancer.

2. Etravirine for the use as claimed in claim 1, wherein the cancer is a solid tumor or a hematologic malignancy.

3. Etravirine for the use as claimed in claim 2, wherein the cancer is a solid tumor selected from colorectal carcinoma, pancreatic carcinoma, prostate carcinoma, glioblastoma multiforme (GBM), non-small-cell lung carcinoma (NSCLC), and neuroblastoma.

4. Etravirine for the use as claimed in any one of claims 1 to 3, wherein the subject to be treated has a solid tumor exhibiting over-expression, activation, or amplification of a kinase selected from Phosphoinositide 3-Kinase (PI3K), e.g. from the isoforms p120γ (PI3Kγ), p110β/p85α (PI3Kβ), p110δ/p85α (PI3Kδ), and p110α/p85α (PI3Kα), Janus Kinase 2 (JAK2), Aurora Kinase A (Aurora A), Proto-oncogene Serine/Threonine-Protein Kinase Pim-1 (Pim1), Cyclin-Dependent Kinase 2/Cyclin E Complex (CDK2/Cyclin E), Cyclin-Dependent Kinase 6/Cyclin D3 Complex (CDK6/Cyclin D3), Mechanistic Target of Rapamycin (mTOR) and Cyclin-Dependent Kinase 1/Cyclin A Complex (CDK1/Cyclin A).

5. Etravirine for the use as claimed in claim 4, wherein subject to be treated has a dysregulation in the PI3K/AKT/mTOR pathway, JAK/STAT pathway, or cell cycle regulatory pathways.

6. Etravirine for the use as claimed in any one of claims 1 to 5, wherein the cancer is resistant to standard therapies and/or associated with a poor prognosis.

7. A pharmaceutical composition comprising Etravirine and a pharmaceutical excipient for use in the treatment or prevention of cancer, preferably a solid tumor.

8. The pharmaceutical composition for use as claimed in claim 7, wherein the pharmaceutical excipient is selected from diluents, binders, disintegrants, glidants and lubricants.

9. The pharmaceutical composition for use as claimed in claim 7 or 8, wherein the pharmaceutical composition is a tablet.

10. The pharmaceutical composition for use as claimed in any one of claims 7 to 9, wherein the pharmaceutical composition contains 25 mg, 100 mg or 200 mg Etravirine.

11. The pharmaceutical composition for use as claimed in any one of claims 7 to 10, wherein Etravirine is to be administered in a daily dose of 100 mg to 800 mg, preferably 200 mg to 400 mg.

12. The pharmaceutical composition for use as claimed in any one of claims 7 to 11, wherein the pharmaceutical composition is to be administered separately, concurrently, or sequentially with another anti-cancer agent.

13. The pharmaceutical composition for use as claimed in claim 12, wherein the other anti-cancer agent is a chemotherapeutic agent, targeted therapy, or a kinase inhibitor.
